# EUROPÄISCHE PATENTSCHRIFT

(11) **EP 4 275 653 B1**
(45) Veröffentlichungstag und Bekanntmachung des Hinweises auf die Patenterteilung: **25.02.2026**
(21) Anmeldenummer: 23169846.5
(22) Anmeldetag: 25.04.2023
(51) Int. Cl.: A61F 2/16

(54) **INTRAOKULARLINSE MIT EINER AUFSPANNVORRICHTUNG**
INTRAOCULAR LENS WITH A CLAMPING DEVICE
LENTILLE INTRAOCULAIRE AVEC DISPOSITIF DE SERRAGE

(30) Priorität: 10.05.2022 DE 102022111612
(43) Veröffentlichungstag der Anmeldung: 15.11.2023
(73) Patentinhaber: Carl Zeiss Meditec AG, 07745 Jena (DE)
(72) Erfinder: MASCH, Jennifer-Magdalena, 73447 Oberkochen (DE)
(74) Vertreter: PATERIS Patentanwälte PartmbB

(56) Entgegenhaltungen:
- EP-B1- 2 536 352
- EP-B1- 2 846 735
- US-A1- 2005 107 875

## Beschreibung

Die Erfindung betrifft eine Intraokularlinse mit einer Aufspannvorrichtung.

Bei einer Kataraktbehandlung wird eine natürliche Linse des menschlichen Auges durch eine künstliche Intraokularlinse ersetzt. Dazu wird die natürliche Linse beispielsweise mittels Phakoemulsifikation zerkleinert und anschließend aus einem Kapselsack des Auges abgesaugt. Bei einer extrakapsulären Katarakt-Extraktion verbleibt eine Linsenkapsel der Linse dabei in dem Kapselsack. Die Intraokularlinse hat in der Regel eine im Wesentlichen planare Form. Dadurch kann die Linsenkapsel in einem hinteren Bereich des Kapselsacks die Intraokularlinse berühren. Dies kann dazu führen, dass mehrere Monate oder sogar Jahre nach der Kataraktbehandlung ein sogenannter Nachstar entstehen kann, der zu einer Trübung der Linsenkapsel in dem hinteren Bereich des Kapselsacks führt. Die Trübung geht einher mit einer Verschlechterung des Sehens. Der Nachstar kann durch eine sogenannte Kapsulotomie behandelt werden. Dabei kann die Trübung beispielsweise mit mehreren Impulsen von Laserstrahlung beaufschlagt werden. Wünschenswert wäre es jedoch, wenn der Nachstar nach der Kataraktbehandlung gar nicht erst entsteht. US 2010 / 0 228 344 A1 offenbart eine verformbare intrakapsuläre Implantationsvorrichtung zum Verformen eines entkernten Kapselsackes. EP 2 536 352 B1 offenbart eine akkommodierende Intraokularlinse. EP 2 846 735 B1 offenbart eine akkommodierende-disakkommodierende Intraokularlinse. US 2005/0107875 A1 offenbart eine akkommodierende Linse mit Haptiken.

Aufgabe der Erfindung ist es daher, eine Intraokularlinse zu schaffen, bei der die Wahrscheinlichkeit eines Entstehens eines Nachstars nach einer Kataraktbehandlung gering ist.

Eine erste erfindungsgemäße Intraokularlinse weist einen Optikkörper, der eine optische Achse aufweist, und eine Haptik auf, die einen Haptikarm und eine Aufspannvorrichtung aufweist. Der Haptikarm ist an dem Optikkörper befestigt sowie weist ein erstes Längsende, das dem Optikkörper zugewandt angeordnet ist, und ein zweites Längsende auf, das dem Optikkörper abgewandt angeordnet ist. Die Aufspannvorrichtung ist an dem zweiten Längsende befestigt, weist einen Formgedächtniswerkstoff mit einer Übergangstemperatur und ein Längsende auf. Zudem hat die Aufspannvorrichtung einen aufgespannten Zustand, in dem das Längsende derjenige Teil der Oberfläche der Aufspannvorrichtung ist, der in einer Richtung senkrecht zu einer Radialrichtung bezüglich der optischen Achse am weitesten von dem Haptikarm entfernt ist, und einen nicht aufgespannten Zustand, in dem das Längsende in der Richtung senkrecht zu der Radialrichtung weniger weit von dem Haptikarm als in dem aufgespannten Zustand entfernt ist, wobei die Aufspannvorrichtung eingerichtet ist, durch ein Überschreiten der Übergangstemperatur von dem nicht aufgespannten Zustand in den aufgespannten Zustand zu gelangen.

Eine zweite erfindungsgemäße Intraokularlinse weist einen Optikkörper, der eine optische Achse aufweist, und eine Haptik auf, die einen Haptikarm und eine Aufspannvorrichtung aufweist. Der Haptikarm ist an dem Optikkörper befestigt sowie weist ein erstes Längsende, das dem Optikkörper zugewandt angeordnet ist, und ein zweites Längsende auf, das dem Optikkörper abgewandt angeordnet ist. Die Aufspannvorrichtung ist an dem zweiten Längsende befestigt, weist einen pseudoelastischen Werkstoff und ein Längsende auf. Zudem hat die Aufspannvorrichtung einen aufgespannten Zustand, in dem das Längsende derjenige Teil der Oberfläche der Aufspannvorrichtung ist, der in einer Richtung senkrecht zu einer Radialrichtung bezüglich der optischen Achse am weitesten von dem Haptikarm entfernt ist, und einen nicht aufgespannten Zustand, in dem das Längsende in der Richtung senkrecht zu der Radialrichtung weniger weit von dem Haptikarm als in dem aufgespannten Zustand entfernt ist, wobei die Aufspannvorrichtung eingerichtet ist, dass bei Anwenden einer Kraft auf die Aufspannvorrichtung in Richtung zu dem Haptikarm hin die Aufspannvorrichtung von dem aufgespannten Zustand in den nicht aufgespannten Zustand und nach einem Wegfall der Kraft von dem nicht aufgespannten Zustand in den aufgespannten Zustand gelangt.

In dem aufgespannten Zustand ist die Aufspannvorrichtung sperriger als in dem nicht aufgespannten Zustand. Dadurch kann die Intraokularlinse in dem nicht aufgespannten Zustand leichter durch eine Spitze eines Injektors gedrückt werden, um während einer Kataraktbehandlung in den Kapselsack eines Auges injiziert zu werden. Eine Beschädigung der Aufspannvorrichtung, während die Intraokularlinse durch die Spitze gedrückt wird, ist in dem nicht aufgespannten Zustand unwahrscheinlich. In dem Kapselsack kann die Aufspannvorrichtung in den aufgespannten Zustand gebracht werden. Für die erste erfindungsgemäße Intraokularlinse erfolgt dies, indem der Formgedächtniswerkstoff von einer Temperatur unterhalb der Übergangstemperatur auf eine Temperatur oberhalb der Übergangstemperatur erwärmt wird. Dies kann beispielsweise durch ein Erwärmen mittels Körperwärme, mittels einer Beaufschlagung von elektromagnetischer Strahlung, insbesondere Laserstrahlung, und/oder mittels Beaufschlagung eines alternierenden Magnetfeldes erfolgen. Bei der Beaufschlagung des alternierenden Magnetfeldes kann die Intraokularlinse magnetische Partikel und/oder magnetisierbare Partikel aufweisen, die die Energie des alternierenden Magnetfeldes in Wärme umwandeln. Bei der zweiten erfindungsgemäßen Intraokularlinse kann die Aufspannvorrichtung in den nicht aufgespannten Zustand gebracht werden, indem die Intraokularlinse in die Spitze des Injektors gedrückt wird und somit die Kraft in Richtung zu dem Haptikarm hin auf die Aufspannvorrichtung aufgebracht wird. Nach Verlassen der Spitze fällt diese Kraft weg und die Aufspannvorrichtung gelangt somit in dem Kapselsack in den aufgespannten Zustand. In dem aufgespannten Zustand spannt die Aufspannvorrichtung den Kapselsack auf, wodurch der Kapselsack weiter entfernt von dem Optikkörper angeordnet ist als bei einer herkömmlichen Intraokularlinse, die planar ausgeführt ist. Dadurch kann eine in dem Kapselsack befindliche Flüssigkeit besser als bei der herkömmlichen Intraokularlinse zirkulieren, wodurch eine Wahrscheinlichkeit gering ist, dass sich nach der Kataraktbehandlung ein Nachstar bildet.

Es ist bevorzugt, dass der Formgedächtniswerkstoff eingerichtet ist, durch eine Änderung seiner Krümmung die Aufspannvorrichtung von dem nicht aufgespannten Zustand in den aufgespannten Zustand zu befördern. Insbesondere verringert sich die Krümmung des Formgedächtniswerkstoffs, wenn die Übergangstemperatur überschritten wird.

Die Übergangstemperatur beträgt bevorzugt maximal 35°C. Dadurch ist es möglich, dass die Übergangstemperatur durch eine Erwärmung mittels der Körperwärme überschritten werden kann. Es ist bevorzugt, dass die Übergangstemperatur mindestens 25°C oder mindestens 30°C beträgt. Dadurch kann vermieden werden, dass die Übergangstemperatur während einer Lagerung der Intraokularlinse überschritten wird.

Der Formgedächtniswerkstoff weist bevorzugt ein Polymer auf oder besteht bevorzugt aus dem Polymer. Bei dem Polymer kann es sich beispielsweise um eine Poly(meth)acrylsäure handeln, insbesondere wenn die Intraokularlinse hydrophob ist. Alternativ kann es sich bei dem Polymer um ein 2-Hydoxyethyl-methacrylat und/oder ein 6-Hydroxyhexyl-methacrylat handeln, insbesondere wenn die Intraokularlinse hydrophil ist. Dabei ist besonders bevorzugt, dass die Aufspannvorrichtung aus dem Polymer besteht. Es ist auch denkbar, dass die vollständige Haptik und/oder der Optikkörper aus dem Polymer besteht. Alternativ ist denkbar, dass der Optikkörper und/oder der Haptikarm einen anderen Werkstoff als das Polymer aufweist.

Der Formgedächtniswerkstoff weist bevorzugt eine Legierung auf oder besteht bevorzugt aus der Legierung. Beispielsweise kann eine solche Legierung Nickel und Titan aufweisen. Eine solche Legierung ist unter dem Namen Nitinol bekannt. Besonders bevorzugt weist die Aufspannvorrichtung einen Kunststoff auf, in dem die Legierung eingebracht ist. Bei dem Kunststoff kann es sich beispielsweise um den Werkstoff des Optikkörpers handeln, wie beispielsweise ein Acrylharz. Auch kann es sich bei dem Kunststoff um Polyurethan, Polyvinylidenfluorid und/oder Polytetrafluorethylen handeln. Die Legierung ist besonders bevorzugt beschichtet, insbesondere mit dem Acrylharz, dem Polyurethan, dem Polyvinylidenfluorid und/oder dem Polytetrafluorethylen. Auch ist es denkbar, die Legierung mit Phosphorylcholin zu behandeln, um die Legierung zu beschichten.

Der pseudoelastische Werkstoff kann ein Formgedächtniswerkstoff, insbesondere eine Formgedächtnislegierung, sein, bei dem zusätzlich zur gewöhnlichen elastischen Verformung eine durch äußere Krafteinwirkung verursachte reversible Formänderung beobachtet wird. Der pseudoelastische Werkstoff ist bevorzugt eingerichtet, durch eine Änderung seiner Krümmung die Aufspannvorrichtung von dem nicht aufgespannten Zustand in den aufgespannten Zustand zu befördern. Beispielsweise kann es sich bei dem pseudoelastischen Werkstoff um Nitinol handeln. Beispielsweise kann sich die Krümmung des pseudoelastischen Werkstoffs verringern, wenn die Aufspannvorrichtung von dem nicht aufgespannten Zustand in den aufgespannten Zustand gelangt.

Es ist für die erste erfindungsgemäße Intraokularlinse bevorzugt und für die zweite erfindungsgemäße Intraokularlinse erfindungsgemäß, dass die Aufspannvorrichtung in dem nicht aufgespannten Zustand sich vollumfänglich um den Haptikarm erstreckt und die Aufspannvorrichtung einen Rand aufweist, der geschlossen ausgebildet ist und in dem aufgespannten Zustand das in der Richtung senkrecht zu der Radialrichtung außenliegende Ende der Aufspannvorrichtung ist. Durch das Vorsehen des geschlossen ausgebildeten Rands entfernt sich die Aufspannvorrichtung entlang des gesamten Umfangs um den Haptikarm von dem Haptikarm wie bei einem Regenschirm, wenn die Aufspannvorrichtung von dem nicht aufgespannten Zustand in den aufgespannten Zustand gelangt. Dadurch kann der Kapselsack besonders weit aufgespannt werden. Dabei ist besonders bevorzugt, dass in dem aufgespannten Zustand der Rand die Form eines Kreises hat.

Die Aufspannvorrichtung weist bevorzugt mehrere der Längsenden auf. Dadurch kann der Kapselsack besonders weit aufgespannt werden.

Es ist bevorzugt, dass die Intraokularlinse mindestens zwei der Haptiken aufweist. Dadurch kann der Kapselsack besonders weit aufgespannt werden. Die Anzahl der Haptiken ist besonders bevorzugt gerade, insbesondere ist die Anzahl der Haptiken 2 oder 4.

Im Folgenden wird anhand der beigefügten schematischen Zeichnungen die Erfindung näher erläutert. Es zeigen
Figur 1 einen Schnitt durch eine erste Ausführungsform einer erfindungsgemäßen Intraokularlinse mit einer Aufspannvorrichtung, die sich in einem nicht aufgespannten Zustand befindet,
Figur 2 den Schnitt aus Figur 1, wobei die Aufspannvorrichtung sich in dem aufgespannten Zustand befindet,
Figur 3 eine Seitenansicht einer zweiten Ausführungsform der Intraokularlinse,
Figur 4 eine Seitenansicht einer dritten Ausführungsform der Intraokularlinse,
Figur 5 eine Seitenansicht einer ersten Ausführungsform einer Haptik der Intraokularlinse,
Figur 6 eine Ansicht von oben auf die erste Ausführungsform der Haptik,
Figur 7 eine Seitenansicht einer zweiten Ausführungsform einer Haptik der Intraokularlinse,
Figur 8 eine Ansicht von oben auf die zweite Ausführungsform der Haptik,
Figur 9 eine Seitenansicht einer dritten Ausführungsform einer Haptik der Intraokularlinse,
Figur 10 eine Ansicht von oben auf die dritte Ausführungsform der Haptik,
Figur 11 eine Seitenansicht einer vierten Ausführungsform einer Haptik der Intraokularlinse,
Figur 12 eine Ansicht von oben auf die vierte Ausführungsform der Haptik.

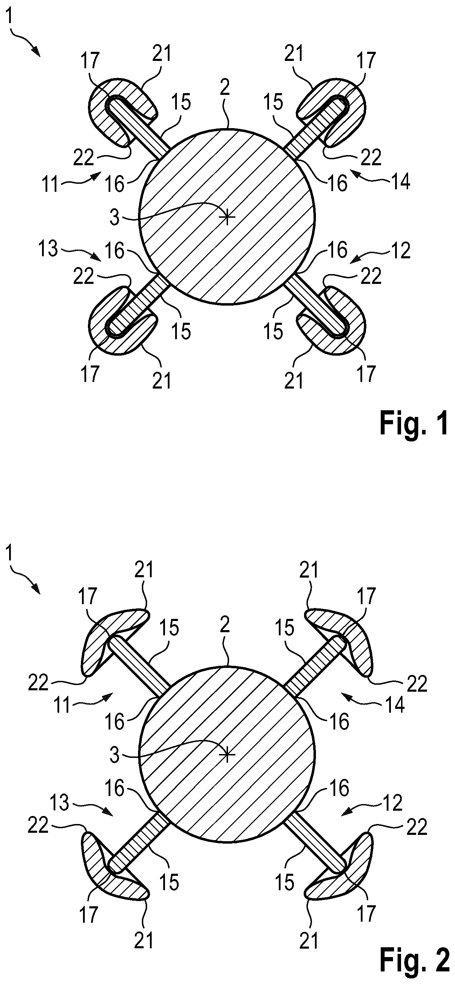

Wie es aus Figuren 1 bis 4 ersichtlich ist, weist eine Intraokularlinse 1 einen Optikkörper 2, der eine optische Achse 3 aufweist, und eine Haptik 11 bis 14 auf, die einen Haptikarm 15 und eine Aufspannvorrichtung 21 aufweist. Der Haptikarm 15 ist an dem Optikkörper 2 befestigt sowie weist ein erstes Längsende 16, das dem Optikkörper 2 zugewandt angeordnet ist, und ein zweites Längsende 17 auf, das dem Optikkörper 2 abgewandt angeordnet ist. Die Aufspannvorrichtung 21 ist an dem zweiten Längsende 17 befestigt und weist ein Längsende 22 auf. Die Aufspannvorrichtung 21 hat einen aufgespannten Zustand (siehe Figur 2), in dem das Längsende 22 derjenige Teil der Oberfläche der Aufspannvorrichtung 21 ist, der in einer Richtung senkrecht zu einer Radialrichtung 6 bezüglich der optischen Achse 3 am weitesten von dem Haptikarm 15 entfernt ist, und einen nicht aufgespannten Zustand (siehe Figur 2), in dem das Längsende 22 in der Richtung senkrecht zu der Radialrichtung 6 weniger weit von dem Haptikarm 15 als in dem aufgespannten Zustand entfernt ist.

Dabei ist denkbar, dass die Aufspannvorrichtung 21 einen Formgedächtniswerkstoff mit einer Übergangstemperatur aufweist und dass die Aufspannvorrichtung 21 eingerichtet ist, durch ein Überschreiten der Übergangstemperatur von dem nicht aufgespannten Zustand in den aufgespannten Zustand zu gelangen. Die Übergangstemperatur kann beispielsweise in einem Bereich von 25°C bis 35°C oder von 30°C bis 35°C liegen. Alternativ ist denkbar, dass die Aufspannvorrichtung 21 einen pseudoelastischen Werkstoff aufweist und dass die Aufspannvorrichtung 21 eingerichtet ist, dass bei Anwenden einer Kraft auf die Aufspannvorrichtung 21 in Richtung zu dem Haptikarm 15 hin die Aufspannvorrichtung 21 von dem aufgespannten Zustand in den nicht aufgespannten Zustand und nach einem Wegfall der Kraft von dem nicht aufgespannten Zustand in den aufgespannten Zustand gelangt.

Ein Vergleich von Figur 1 mit Figur 2 zeigt, dass der Formgedächtniswerkstoff eingerichtet sein kann, durch eine Verringerung seiner Krümmung die Aufspannvorrichtung 21 von dem nicht aufgespannten Zustand in den aufgespannten Zustand zu befördern. In analoger Weise kann der pseudoelastische Werkstoff eingerichtet sein, durch eine Verringerung seiner Krümmung die Aufspannvorrichtung 21 von dem nicht aufgespannten Zustand in den aufgespannten Zustand zu befördern.

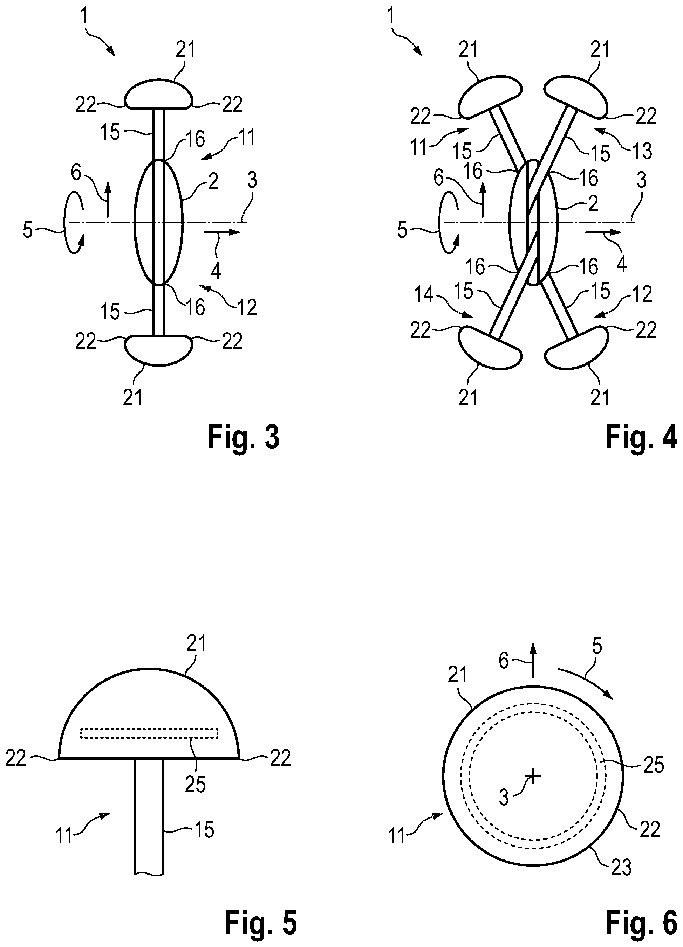

Figuren 1 bis 4 zeigen, dass die Intraokularlinse 1 mindestens zwei der Haptiken 11 bis 14 aufweisen kann. Die Haptiken 11 bis 14 können beispielsweise in einer Umfangsrichtung 5 bezüglich der optischen Achse 3 gleichmäßig um den Optikkörper 2 herum angeordnet sein. Es ist denkbar, dass die Anzahl der Haptiken 11 bis 14 gerade ist, insbesondere kann die Anzahl der Haptiken zwei (siehe Figur 3) oder vier (siehe Figuren 1, 2 und 4) sein.

Die Haptikarme 15 können beispielsweise die Form eines Stabes haben, vergleiche Figuren 1 bis 12. Insbesondere können die Haptikarme 15 beispielsweise in einer Radialrichtung 6 bezüglich der optischen Achse 3 orientiert sein, vergleiche Figur 3. Alternativ ist denkbar, dass die Haptikarme 15 zu der optischen Achse 3 hin geneigt sind, vergleiche Figur 4. Dabei ist denkbar, dass die Haptikarme 15 in der Umfangsrichtung 5 abwechselnd in entgegen gesetzte Richtungen geneigt sind. Dadurch kann der Kapselsack besonders weit aufgespannt werden.

Figuren 5 bis 12 zeigen verschiedenen Ausführungsformen für die Haptik 11, wobei die Aufspannvorrichtung 21 der Haptik 11 jeweils in dem aufgespannten Zustand dargestellt ist. Die Aufspannvorrichtung 21 kann in dem nicht aufgespannten Zustand sich vollumfänglich um den Haptikarm 15 erstrecken und die Aufspannvorrichtung 21 kann einen Rand 23 aufweisen, der geschlossen ausgebildet ist und in dem aufgespannten Zustand das in der Richtung senkrecht zu der Radialrichtung 6 außenliegende Ende der Aufspannvorrichtung 21 ist, vergleiche Figuren 5 bis 10.

Figur 6 zeigt, dass in dem aufgespannten Zustand der Rand 23 die Form eines Kreises haben kann. Der Rand 23 kann symmetrisch zu dem Haptikarm 15 angeordnet sein, so dass das Längsende 22 und der Rand 23 zusammenfallen.

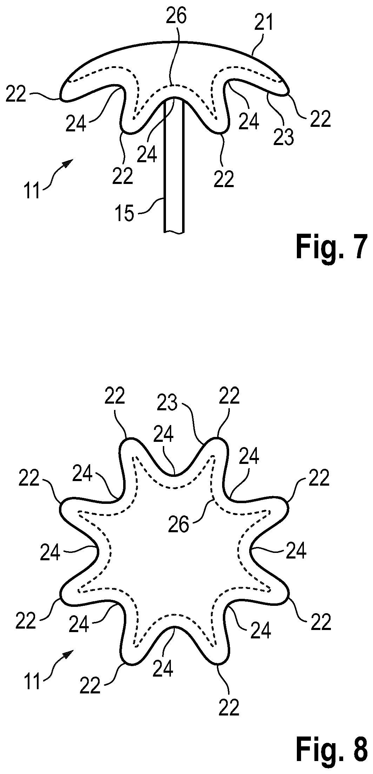

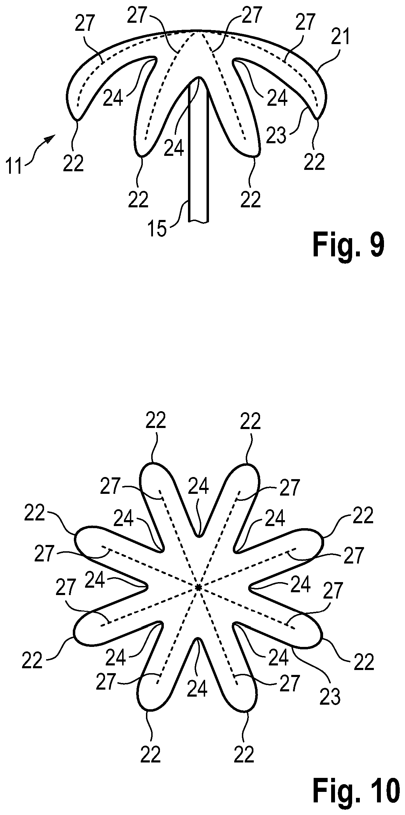

Wie es aus Figuren 7 bis 12 ersichtlich ist, kann die Aufspannvorrichtung 21 mehrere der Längsenden 22 aufweisen. Beispielsweise kann dabei die Aufspannvorrichtung 21 blütenförmig ausgebildet sein, vergleiche Figuren 7 bis 10. Dabei weist der Rand 23 in dem aufgespannten Zustand abwechselnd die Längsenden 22 und Minima 24 auf, die in der Richtung senkrecht zu der Radialrichtung 6 weniger weit von dem Haptikarm 15 als die Längsenden 22 entfernt sind. Bei der Ausführungsform gemäß Figuren 9 und 10 sind die Minima 24 deutlich ausgeprägter als bei der Ausführungsform gemäß Figuren 7 und 8. Der Abstand von dem Haptikarm 15 zu den Minima 24 kann in einem Bereich von 20% bis 90%, bevorzugt von 40% bis 60 %, des Abstandes des Haptikarms 15 zu den Längsenden 22 betragen.

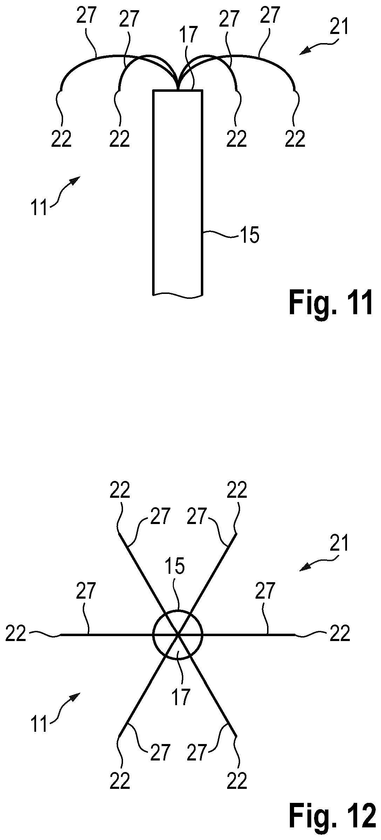

Figuren 5 bis 12 zeigen, dass der Formgedächtniswerkstoff und/oder der pseudoelastische Werkstoff ein Metall oder eine Legierung aufweisen kann. In Figuren 5 bis 10 ist der Formgedächtniswerkstoff und/oder der pseudoelastische Werkstoff in einen Kunststoff eingebracht. Bei der Ausführungsform der Haptik 11 gemäß Figuren 5 und 6 hat der Formgedächtniswerkstoff und/oder der pseudoelastische Werkstoff die Form eines Rings 25, insbesondere eine Kreisrings. Bei der Ausführungsform der Haptik 11 gemäß Figuren 7 und 8 hat der Formgedächtniswerkstoff und/oder der pseudoelastische Werkstoff eine geschlossene Wellenform 26. Bei den Ausführungsform der Haptik 11 gemäß Figuren 9 bis 12 liegt der Formgedächtniswerkstoff und/oder der pseudoelastische Werkstoff in Form von mehreren Drähten 27 vor. In dem Fall, dass die Anzahl der Längsenden 22 gerade ist, erstreckt sich jeder der Drähte 27 jeweils von einem der Längsenden 22 zu einem anderen der Längsenden 22. In dem Fall, dass die Anzahl der Längsenden 22 ungerade ist, erstreckt sich jeder der Drähte 27 ausgehend von dem zweiten Längsende 17 des Haptikarms 15 bis zu jeweils einem der Längsenden 22. Bei der Ausführungsform gemäß Figuren 11 und 12 ist denkbar, dass der Formgedächtniswerkstoff und/oder der pseudoelastische Werkstoff beschichtet ist, um dadurch eine gute Biokompatibilität zu erreichen.

Alternativ dazu, in den Ausführungsformen gemäß Figuren 5 bis 12 das Metall oder die Legierung vorzusehen, ist es auch denkbar, dass der Formgedächtniswerkstoff ein Polymer ist. Insbesondere kann die Aufspannvorrichtung aus dem Polymer bestehen. Bei dem Polymer kann es sich beispielsweise um eine Poly(meth)acrylsäure handeln, insbesondere wenn die Intraokularlinse hydrophob ist. Alternativ kann es sich bei dem Polymer um ein 2-Hydoxyethyl-methacrylat und/oder ein 6-Hydroxyhexyl-methacrylat handeln, insbesondere wenn die Intraokularlinse hydrophil ist.

### Bezugszeichenliste

1 Intraokularlinse
2 Optikkörper
3 optische Achse
4 Axialrichtung
5 Umfangsrichtung
6 Radialrichtung
11 erste Haptik
12 zweite Haptik
13 dritte Haptik
14 vierte Haptik
15 Haptikarm
16 erstes Längsende des Haptikarms
17 zweites Längsende des Haptikarms
21 Aufspannvorrichtung
22 Längsende
23 Rand
24 Minimum
25 Ring
26 geschlossene Wellenform
27 Draht

## Patentansprüche

1. Intraokularlinse mit einem Optikkörper (2), der eine optische Achse (3) aufweist, und einer Haptik (11 bis 14), die einen Haptikarm (15) aufweist, der an dem Optikkörper (2) befestigt ist sowie ein erstes Längsende (16), das dem Optikkörper (2) zugewandt angeordnet ist, und ein zweites Längsende (17) aufweist, das dem Optikkörper (2) abgewandt angeordnet ist, **dadurch gekennzeichnet, dass** die Haptik (11 bis 14) eine Aufspannvorrichtung (21) aufweist, die an dem zweiten Längsende (17) befestigt ist, einen Formgedächtniswerkstoff mit einer Übergangstemperatur aufweist und ein Längsende (22) aufweist, wobei die Aufspannvorrichtung (21) einen aufgespannten Zustand, in dem das Längsende (22) derjenige Teil der Oberfläche der Aufspannvorrichtung (21) ist, der in einer Richtung senkrecht zu einer Radialrichtung (6) bezüglich der optischen Achse (3) am weitesten von dem Haptikarm (15) entfernt ist, und einen nicht aufgespannten Zustand hat, in dem das Längsende (22) in der Richtung senkrecht zu der Radialrichtung (6) weniger weit von dem Haptikarm (15) als in dem aufgespannten Zustand entfernt ist, wobei die Aufspannvorrichtung (21) eingerichtet ist, durch ein Überschreiten der Übergangstemperatur von dem nicht aufgespannten Zustand in den aufgespannten Zustand zu gelangen.

2. Intraokularlinse gemäß Anspruch 1, wobei der Formgedächtniswerkstoff eingerichtet ist, durch eine Änderung seiner Krümmung die Aufspannvorrichtung (21) von dem nicht aufgespannten Zustand in den aufgespannten Zustand zu befördern.

3. Intraokularlinse gemäß Anspruch 1 oder 2, wobei die Übergangstemperatur maximal 35°C und insbesondere mindestens 25°C oder mindestens 30°C beträgt.

4. Intraokularlinse gemäß einem der Ansprüche 1 bis 3, wobei der Formgedächtniswerkstoff ein Polymer ist.

5. Intraokularlinse gemäß einem der Ansprüche 1 bis 3, wobei der Formgedächtniswerkstoff eine Legierung ist.

6. Intraokularlinse mit einem Optikkörper (2), der eine optische Achse (3) aufweist, und einer Haptik (11 bis 14), die einen Haptikarm (15) aufweist, der an dem Optikkörper (2) befestigt ist sowie ein erstes Längsende (16), das dem Optikkörper (2) zugewandt angeordnet ist, und ein zweites Längsende (17) aufweist, das dem Optikkörper (2) abgewandt angeordnet ist, **dadurch gekennzeichnet, dass** die Haptik (11 bis 14) eine Aufspannvorrichtung (21) aufweist, die an dem zweiten Längsende (17) befestigt ist, einen pseudoelastischen Werkstoff und ein Längsende (22) aufweist, wobei die Aufspannvorrichtung (21) einen aufgespannten Zustand, in dem das Längsende (22) derjenige Teil der Oberfläche der Aufspannvorrichtung (21) ist, der in einer Richtung senkrecht zu einer Radialrichtung (6) bezüglich der optischen Achse (3) am weitesten von dem Haptikarm (15) entfernt ist, und einen nicht aufgespannten Zustand hat, in dem das Längsende (22) in der Richtung senkrecht zu der Radialrichtung (6) weniger weit von dem Haptikarm (15) als in dem aufgespannten Zustand entfernt ist, wobei die Aufspannvorrichtung (21) eingerichtet ist, dass bei Anwenden einer Kraft auf die Aufspannvorrichtung (21) in Richtung zu dem Haptikarm (15) hin die Aufspannvorrichtung (21) von dem aufgespannten Zustand in den nicht aufgespannten Zustand und nach einem Wegfall der Kraft von dem nicht aufgespannten Zustand in den aufgespannten Zustand gelangt, wobei die Aufspannvorrichtung (21) in dem nicht aufgespannten Zustand sich vollumfänglich um den Haptikarm (15) erstreckt und die Aufspannvorrichtung (21) einen Rand (23) aufweist, der geschlossen ausgebildet ist und in dem aufgespannten Zustand das in der Richtung senkrecht zu der Radialrichtung (6) außenliegende Ende der Aufspannvorrichtung (21) ist.

7. Intraokularlinse gemäß Anspruch 6, wobei der pseudoelastische Werkstoff eingerichtet ist, durch eine Änderung seiner Krümmung die Aufspannvorrichtung (21) von dem nicht aufgespannten Zustand in den aufgespannten Zustand zu befördern.

8. Intraokularlinse gemäß Anspruch 6 oder 7, wobei in dem aufgespannten Zustand der Rand (23) die Form eines Kreises hat.

9. Intraokularlinse gemäß einem der Ansprüche 1 bis 8, wobei die Aufspannvorrichtung (21) mehrere der Längsenden (22) aufweist.

## Claims

1. Intraocular lens having an optic (2) with an optical axis (3) and having a haptic (11 to 14) which comprises a haptic arm (15) that is secured to the optic (2) and has a first longitudinal end (16) arranged close to the optic (2) and a second longitudinal end (17) arranged away from the optic (2), **characterized in that** the haptic (11 to 14) comprises a deployment device (21), which is secured to the second longitudinal end (17), comprises a shape memory material with a transition temperature and comprises a longitudinal end (22), wherein the deployment device (21) has a deployed state, in which the longitudinal end (22) is that part of the surface of the deployment device (21) which is furthest away from the haptic arm (15) with respect to the optical axis (3) in a direction perpendicular to a radial direction (6), and a non-deployed state, in which the longitudinal end (22) is not as far away from the haptic arm (15) as in the deployed state in the direction perpendicular to the radial direction (6), wherein the deployment device (21) is configured to transition to the deployed state from the non-deployed state by exceeding the transition temperature.

2. Intraocular lens according to Claim 1, wherein the shape memory material is configured to convey the deployment device (21) from the non-deployed state to the deployed state by changing its curvature.

3. Intraocular lens according to Claim 1 or 2, wherein the transition temperature is no more than 35°C and in particular at least 25°C or at least 30°C.

4. Intraocular lens according to any of Claims 1 to 3, wherein the shape memory material is a polymer.

5. Intraocular lens according to any of Claims 1 to 3, wherein the shape memory material is an alloy.

6. Intraocular lens having an optic (2) with an optical axis (3) and having a haptic (11 to 14) which comprises a haptic arm (15) that is secured to the optic (2) and has a first longitudinal end (16) arranged close to the optic (2) and a second longitudinal end (17) arranged away from the optic (2), **characterized in that** the haptic (11 to 14) comprises a deployment device (21), which is secured to the second longitudinal end (17), comprises a pseudoelastic material and comprises a longitudinal end (22), wherein the deployment device (21) has a deployed state, in which the longitudinal end (22) is that part of the surface of the deployment device (21) which is furthest away from the haptic arm (15) with respect to the optical axis (3) in a direction perpendicular to a radial direction (6), and a non-deployed state, in which the longitudinal end (22) is not as far away from the haptic arm (15) as in the deployed state in the direction perpendicular to the radial direction (6), wherein the deployment device (21) is configured such that when a force is applied to the deployment device (21) in the direction towards the haptic arm (15), the deployment device (21) transitions to the non-deployed state from the deployed state and, after the force ceases, said deployment device transitions to the deployed state from the non-deployed state, wherein the deployment device (21) extends completely around the haptic arm (15) in the non-deployed state, and the deployment device (21) comprises an edge (23) which has closed form and which in the deployed state is the outer end of the deployment device (21) in the direction perpendicular to the radial direction (6).

7. Intraocular lens according to Claim 6, wherein the pseudoelastic material is configured to convey the deployment device (21) from the non-deployed state to the deployed state by changing its curvature.

8. Intraocular lens according to Claim 6 or 7, wherein the edge (23) has the shape of a circle in the deployed state.

9. Intraocular lens according to any of Claims 1 to 8, wherein the deployment device (21) comprises several of the longitudinal ends (22).

## Revendications

1. Lentille intraoculaire présentant un corps optique (2), qui présente un axe optique (3), et une haptique (11 à 14), qui présente un bras haptique (15), qui est fixé au corps optique (2) et qui présente une première extrémité longitudinale (16), qui est agencée face au corps optique (2), et une seconde extrémité longitudinale (17), qui est agencée à l'opposé du corps optique (2), **caractérisée en ce que** l'haptique (11 à 14) présente un mécanisme tendeur (21), qui est fixé à la seconde extrémité longitudinale (17), qui présente un matériau à mémoire de forme présentant une température de transition et qui présente une extrémité longitudinale (22), le mécanisme tendeur (21) possédant un état tendu, dans lequel l'extrémité longitudinale (22) est la partie de la surface du mécanisme tendeur (21) qui est la plus éloignée du bras haptique (15) dans une direction perpendiculaire à une direction radiale (6) par rapport au bras optique (3), et un état non tendu, dans lequel extrémité longitudinale (22) est moins éloignée du bras haptique (15) que dans l'état tendu dans la direction perpendiculaire à la direction radiale (6), le mécanisme tendeur (21) étant conçu pour passer de l'état non tendu à l'état tendu par un dépassement de la température de transition.

2. Lentille intraoculaire selon la revendication 1, le matériau à mémoire de forme étant conçu pour amener le mécanisme tendeur (21) de son état non tendu dans son état tendu par une modification de sa courbure.

3. Lentille intraoculaire selon la revendication 1 ou 2, la température de transition représentant au maximum 35°C et en particulier au moins 25°C ou au moins 30°C.

4. Lentille intraoculaire selon l'une des revendications 1 à 3, le matériau à mémoire de forme étant un polymère.

5. Lentille intraoculaire selon l'une des revendications 1 à 3, le matériau à mémoire de forme étant un alliage.

6. Lentille intraoculaire présentant un corps optique (2), qui présente un axe optique (3), et une haptique (11 à 14), qui présente un bras haptique (15), qui est fixé au corps optique (2) et qui présente une première extrémité longitudinale (16), qui est agencée face au corps optique (2), et une seconde extrémité longitudinale (17), qui est agencée à l'opposé du corps optique (2), **caractérisée en ce que** l'haptique (11 à 14) présente un mécanisme tendeur (21), qui est fixé à la seconde extrémité longitudinale (17), qui présente un matériau pseudoélastique et une extrémité longitudinale (22), le mécanisme tendeur (21) possédant un état tendu, dans lequel l'extrémité longitudinale (22) est la partie de la surface du mécanisme tendeur (21) qui est la plus éloignée du bras haptique (15) dans une direction perpendiculaire à une direction radiale (6) par rapport au bras optique (3), et un état non tendu, dans lequel extrémité longitudinale (22) est moins éloignée du bras haptique (15) que dans l'état tendu dans la direction perpendiculaire à la direction radiale (6), le mécanisme tendeur (21) étant conçu de telle sorte que lors de l'application d'une force sur le mécanisme tendeur (21) en direction du bras haptique (15), le mécanisme tendeur (21) passe de l'état tendu dans l'état non tendu et, après la suppression de la force, passe de l'état non tendu dans l'état tendu, le mécanisme tendeur (21) s'étendant, dans l'état non tendu, complètement autour du bras haptique (15) et le mécanisme tendeur (21) présentant un bord (23), qui est conçu sous forme fermée et qui est, dans l'état tendu, l'extrémité du mécanisme tendeur (21) qui se trouve à l'extérieur dans la direction perpendiculaire à la direction radiale (6).

7. Lentille intraoculaire selon la revendication 6, le matériau pseudoélastique étant conçu pour amener le mécanisme tendeur (21) de son état non tendu dans son état tendu par une modification de sa courbure.

8. Lentille intraoculaire selon la revendication 6 ou 7, le bord (23) ayant la forme d'un cercle dans l'état tendu.

9. Lentille intraoculaire selon l'une des revendications 1 à 8, le mécanisme tendeur (21) présentant plusieurs extrémités longitudinales (22).
